# DEMANDE DE BREVET EUROPEEN

(11) **EP 1 169 970 A1**
(43) Date de publication de la demande: **09.01.2002**
(21) Numéro de dépôt: 01401771.9
(22) Date de dépôt: 03.07.2001
(51) Int. Cl.: A61B 17/22

(54) **Dispositif d'administration d'une composition dans un conduit du corps humain ou animal**

(30) Priorité: 04.07.2000 FR 0008751; 09.03.2001 FR 0103286
(71) Demandeur: TRANSGENE S.A., 67000 Strasbourg (FR)
(72) Inventeur: Camenzind, Edoardo, 1206 Genève (CH); Neuville, Pascal, 67000 Strasbourg (FR); Fontao, Lionel, 74100 Annemasse (FR); Calenda, Valérie, 67000 Strasbourg (FR)
(74) Mandataire: Warcoin, Jacques

(57) **Abrégé**

Dispositif (2) pour administrer une composition dans un conduit (14) du corps humain ou animal comprenant des moyens (4) aptes à entamer une face interne de la paroi du conduit pour réaliser des ouvertures borgnes dans une épaisseur de la paroi et des moyens de distribution (20) pour mettre la composition en contact avec les ouvertures.

## Description

L'invention concerne une méthode et un dispositif à mettre en oeuvre dans le cadre de l'administration dans la paroi d'un conduit du corps humain ou animal d'une composition, notamment dans le cadre d'un traitement ou de la prévention de l'athérosclérose, en particulier pour lutter contre la resténose faisant suite à la pose d'un stent dans un vaisseau sanguin, notamment une artère.

L'athérosclérose (Ross, 1999, Am. Heart. J. 138, 419-20) est une pathologie des artères caractérisée par l'envahissement de l'intima par plusieurs populations cellulaires (cellules musculaires lisses constituant la paroi du vaisseau et cellules inflammatoires) et une accumulation de substances collagéniques et de calcium conduisant à une augmentation de la rigidité de la paroi vasculaire et à une réduction de la lumière artérielle. Une des conséquences les plus graves de cette obstruction des vaisseaux, également appelée sténose, touche les artères coronaires dont le rôle est l'irrigation du coeur. Appelée insuffisance coronarienne, cette atteinte provoque une ischémie myocardique dont le syndrome associé le plus fréquent est l'infarctus (Roberts, 1998, Am. J. Cardiol. 82, 41T-44T).

Deux modes de traitement des sténoses induites par l'athérosclérose sont actuellement proposés aux patients.

Le premier type de traitement, appelé pontage coronarien, se pratique lorsque les sténoses de l'artère sont importantes et multiples (Eagle et al., 1999, J. Am. Coll. Cardiol. 34, 1262-347). Il s'agit d'une approche chirurgicale qui vise à rétablir la circulation sanguine jusqu'au myocarde en contournant la coronaire obstruée. Pour cela, un segment vasculaire prélevé soit sur l'artère mammaire, soit sur la veine saphène est placé en amont et débouche en aval de la partie sténosée. Ce traitement lourd, nécessitant l'ouverture de la cage thoracique, ne se pratique que dans un nombre de cas limité et plus particulièrement lorsque la seconde approche s'avère inapplicable.

La seconde approche, appelée angioplastie coronaire percutanée, consiste, dans un premier temps, à introduire dans la coronaire, au niveau de la zone obstruée, un cathéter muni à son extrémité d'un ballonnet. Dans un deuxième temps, le ballon est gonflé *in situ* afin d'écraser la plaque athéromateuse contre la paroi vasculaire et de rétablir un calibre coronarien suffisant pour permettre une perfusion myocardique satisfaisante(Cishek and Gershony, 1996, Am. Heart. J. 131, 1012-7). Cette seconde technique est la plus utilisée chez les patients souffrant d'insuffisance coronarienne. Elle représente 50 000 interventions par an en France et 500 000 par an aux Etats-Unis. Cependant, le traumatisme infligé à l'artère athéromateuse, lors de la dilatation du ballon, entraîne dans 30% des cas l'apparition au site dilaté d'une nouvelle lésion, appelée resténose (Hong et al., 1997, Curr Probl Cardiol,22,1-36). Cette resténose caractérisée par une nouvelle réduction du calibre de l'artère est en fait liée à la survenue de deux phénomènes successifs. En premier lieu survient un remodelage artériel, qui correspond à une constriction du vaisseau en réponse au phénomène de dilatation et qui s'effectue de façon aiguë dans les heures qui suivent l'intervention (Pasterkamp et al., 2000, Cardiovasc. Res. 45, 843-52). En second lieu, la resténose peut être provoquée par un processus de cicatrisation excessif se caractérisant par une prolifération des cellules musculaires lisses (SMC, pour Smooth Muscular Cells) et une synthèse abondante de matrice extracellulaire (ECM, pour extracellular matrix) qui conduit à une réobstruction symptomatique de la coronaire traitée dans les mois qui suivent l'angioplastie (Schwartz et al., 1996, Int. J. Cardiol. 53, 71-80).

Le phénomène de remodelage peut être maîtrisé par la technique de "stenting" qui consiste en la mise en place, après l'angioplastie, d'une armature, généralement métallique et maillée, appelée stent. Le stent épouse la paroi du vaisseau et confère à l'artère une rigidité artificielle et mécanique qui empêche la phase de constriction aiguë et permet d'obtenir un gain plus important de diamètre artériel. En quelques années, cette procédure s'est généralisée et constitue désormais une technique standard de la cardiologie interventionnelle (Goy and Eeckhout, 1998, Lancet 351, 1943-9).

Toutefois, bien que cette technique permette une amélioration notable du pronostic à court terme des patients traités par angioplastie, des épisodes de resténose surviennent encore chez 30 à 50% des sujets dans les six mois qui suivent la pose du stent.

Il est cependant important de noter que dans ces cas là, la réduction du calibre artériel au niveau du stent est exclusivement liée à une prolifération cellulaire et n'implique pas le phénomène de remodelage artériel. On parlera alors de resténose intra-stent qui est actuellement traitée par redilatation de la zone obstruée à l'aide d'une nouvelle angioplastie. Malheureusement, ce traitement conduit de façon plus fréquente et plus rapide que la première intervention à une re-resténose de la lésion dilatée (Bossi et al., 2000, J. Am. Coll. Cardiol. 35, 1569-76).

L'incidence élevée du phénomène de resténose chez les patients traités par angioplastie et/ou pose de stent constitue un véritable problème de santé publique, responsable d'un coût estimé à 2 milliards de dollars par an aux Etats-Unis. A ce jour, aucun traitement pharmacologique efficace pour la prévention de la resténose, qu'elle soit liée à l'angioplastie et/ou à la pose de stent, n'est encore disponible.

La brachythérapie, reposant sur le positionnement d'un cathéter muni d'une source radioactive au niveau du rétrécissement artériel, permet d'éliminer l'hyperplasie cellulaire (Waksman et al., 2000, Circulation 101, 2165-71). Cependant, cette intervention, qui laisse la paroi non cicatrisée, entraîne des thromboses tardives et s'accompagne d'une prolifération cellulaire aux marges du segment vasculaire irradié (Waksman, 1999, Circulation 100, 780-2). A l'heure actuelle, elle ne constitue pas un traitement satisfaisant.

Une autre approche en cours d'évaluation concerne la mise au point de traitements par thérapie génique. La thérapie génique se définit assez largement comme le transfert d'informations génétiques d'intérêt, dans une cellule ou un organisme hôte. La plupart des stratégies de thérapie génique utilisent des vecteur de transfert pour véhiculer cette information vers et dans la cible cellulaire. De nombreux vecteurs de transfert tant viraux que synthétiques ou plasmidiques ont été développés au cours de ces dernières années et ont fait l'objet de nombreuses publications accessibles à l'homme du métier (voir par exemple Robbins et al, 1988, Tibtech, 16, 34-40 et Rolland, 1998, Therapeutic Drug Carrier Systems, 15, 143-198).

De plus, de nombreuses données expérimentales sont disponibles concernant le transfert de tels vecteurs renfermant des informations génétiques d'intérêt, et particulièrement des gènes, dans des cellules artérielles. On peut, à titre d'exemple, citerles vecteurs adénoviraux qui permettent d'envisager une approche génique de la prévention et/ou du traitement de la resténose. Ainsi, le transfert de gènes codant pour des inhibiteurs de la migration et de la prolifération des cellules musculaires lisses de la paroi artérielle semble une voie thérapeutique prometteuse (Kibbe et al., 2000, Circ. Res. 86, 829-33 ; Macejak et al., 1999, J. Virol. 73, 7745-51 ; Claudio et al., 1999, Circ. Res. 85, 1032-9 ; Perlman et al., 1999, Gene Ther. 6, 758-63). Toutefois, de nombreux problèmes restent à résoudre avant que la thérapie génique intravasculaire n'entre en pratique clinique, et tout particulièrement les problèmes liés à l'efficacité du transfert des vecteurs dans les artères.

En effet, le transfert des vecteurs dans la paroi vasculaire normale ou athéromateuse, et plus particulièrement dans les cellules la constituant, demeure d'une efficacité réduite. Les lames élastiques qui confèrent la plasticité aux artères constituent une barrière génant la pénétration en profondeur des vecteurs de transfert et la présence de plaques athéromateuses calcifiées chez l'individu malade diminue encore l'efficacité de ce transfert (Maillard et al., 1998, Gene Ther. 5, 1023-30 ; Rekhter et al., 1998, Circ. Res. 82, 1243-1252). De même, le tissu resténotique constitué majoritairement de cellules musculaires lisses et de cellules inflammatoires contient une abondante matrice extracellulaire qui constitue une barrière réduisant considérablement le transfert des vecteurs vers et dans les cellules cibles.

En outre, l'administration intra-coronarienne de vecteurs de transfert est rendue difficile par la fonction d'oxygénation du coeur qui est assurée par ces artères. Ainsi, les expériences décrites de thérapie génique réalisées sur des artères carotides ou fémorales de rat et de lapin nécessitent un blocage de la circulation sanguine afin de mettre en contact la composition contenant ledit vecteur de transfert et la paroi vasculaire pendant une durée suffisante pour permettre une efficacité maximale d'administration, et par conséquent de transfert du vecteur dans les cellules,. Une telle approche n'est pas compatible avec la fonction des artères coronaires. Il est en effet impossible de bloquer durablement la circulation dans ces vaisseaux sans provoquer un accident cardiaque grave du à une oxygénation insuffisante. Par conséquent, les temps de contact entre les cellules artérielles des coronaires et la composition renfermant le vecteur de transfert sont nécessairement très courts, ce qui se traduit souvent par une faible efficacité de transfert du vecteur vers et dans les cellules cibles de la paroi du vaisseau traité.

Dans ce contexte, un but de l'invention est de fournir une méthode et un dispositif permettant d'administrer rapidement et efficacement une composition dans la paroi d'un conduit du corps humain ou animal, même dans l'hypothèse où un fluide circule dans ce conduit. Plus particulièrement, un but de l'invention est de permettre d'administrer rapidement et le plus efficacement possible des vecteurs de transfert ou des compositions les contenant, notamment au niveau de cellules cibles localisées dans l'épaisseur de la paroi d'undit conduit. Plus spécialement, cette administration efficace d'undit vecteur ou d'une dite composition se traduit par un transfert efficace dudit vecteur vers ou /et dans lesdites cellules.

En vue de la réalisation de ce but, l'invention concerne en premier lieu une méthode pour administrer une composition dans une paroi d'un conduit d'un corps humain ou animal, caractérisée en ce qu'elle comprend les étapes consistant à :
- entamer une face interne de la paroi du conduit pour réaliser des ouvertures borgnes dans une épaisseur de la paroi ; et
- mettre la composition en contact avec les ouvertures pratiquées dans la paroi.

La méthode pourra être réalisée à l'aide de deux dispositifs séparés (exemple 1), assurant chacun l'une ou l'autre de ces étapes, ou d'un seul dispositif combinant les deux propriétés (exemples 2 et 3), c'est à dire un seul dispositif permettant la réalisation des deux étapes précitées.

Selon un mode de réalisation particulier, l'invention concerne une telle méthode pour le traitement ou la prévention de la resténose ou de la re-resténose, et plus particulièrement lorsque celle-ci se produit au niveau d'un stent. Dans un cas particulier de l'invention, un dit stent aura été mis en place dans ledit conduit humain ou animal après traitement dudit conduit par angioplastie. Selon un mode particulier de réalisation, ladite composition renfermera préférentiellement au moins un vecteur de transfert . Selon un autre mode de réalisation de l'invention, ladite composition renfermera une drogue capable de traiter ou prévenir ladite resténose ou ladite re-resténose.

Ainsi, grâce aux ouvertures entamant l'épaisseur de la paroi, la composition est mise en contact directement avec les cellules de la paroi, et tout particulièrement avec les cellules qui sont localisées dans l'espace situé entre la néointima et la lame élastique. L'administration de cette composition ou des composés compris dans une telle composition est donc efficace, même si la durée du contact est brève, par exemple si un fluide circule dans le conduit.

Dans le cas particulier de l'administration au moyen de l'invention d'un vecteur de transfert, ou d'une composition renfermant un tel vecteur, pour lutter contre la resténose ou la re-resténose d'une artère, on a constaté expérimentalement que l'accessibilité des vecteurs aux cellules de la paroi, ainsi que leur transfert dans les cellules, étaient plus généralisés et s'étendaient plus profondément suivant l'épaisseur de cette paroi, permettant ainsi d'envisager à terme une prévention plus efficace de la resténose ou de la re-resténose.

Dans le cadre de la présente invention, par « entamer » on entend indiquer que des coupes et/ou des perforations et/ou des érosions sont réalisées dans l'épaisseur de la paroi du conduit pour réaliser des ouvertures. « Couper », « cisailler », « trancher », « inciser » ou « sectionner », ainsi que « percer », « trouer » ou « vriller » ou « éroder » ou « élimer » sont des synonymes d' « entamer » dans le cadre de la mise en oeuvre de l'invention. Les « ouvertures » au sens de l'invention sont dites « borgnes » dans la mesure où il ne s'agit pas d'une perforation de part et d'autre de la paroi du conduit. Ces ouvertures peuvent présenter différents aspects, sans limitation particulière quant à leur section ou encore leur orientation. Ainsi, de telles ouvertures pourront avoir l'aspect d'une entaille, de largeur variable (par exemple, de 0.5 à 10 mm, préférentiellement de 2.5 à 5 mm), comme permettent de l'obtenir, par exemple, une lame, un rasoir ou un couteau. De telles ouvertures peuvent également avoir l'aspect d'un trou, d'une piqûre, de diamètre variable (par exemple de 0.05 à 1 mm) comme permettent de l'obtenir, par exemple, une pointe, une pique, un trocart. De telles ouvertures peuvent également avoir l'aspect d'une usure, d'une surface grattée comme permettent de l'obtenir un grattoir, une surface rugueuse, abrasive, par exemple. De telles ouvertures peuvent également avoir un aspect diffus, nécrosé, comme permettent de l'obtenir, par exemple l'action d'un composé chimique, d'un rayonnement localisé adapté. Les ouvertures obtenues selon l'invention peuvent être pratiquées longitudinalement ou transversalement par rapport à l'axe du conduit ; de même, elles peuvent être réalisées invariablement selon un axe radial ou oblique par rapport à l'épaisseur dudit conduit.

Les éléments du dispositif selon l'invention permettant d'obtenir de telles ouvertures peuvent être constitués par différents matériaux tels que par exemple un métal ou un alliage, e.g. un alliage à base de cobalt, de nickel et/ou de titane, certains aciers inoxydables ; un polymère à base par exemple de polypropylène, PEEK, HDPE (high density polyethylene), polysulfone, acétyl, PTFE, FEP, uréthane polycarbonate, polyuréthane, silicone, PTFE, ePTFE ou polyoléfine. Il peut également s'agir de verre, de diamant, de résine... De préférence il s'agira d'un matériau acceptable d'un point de vue biologique.

La méthode selon l'invention pourra présenter en outre au moins l'une quelconque des caractéristiques suivantes :
- on entame la face interne en réalisant des incisions dans la paroi ;
- on réalise les incisions suivant une direction radiale par référence à une direction longitudinale du conduit ;
- préalablement à l'étape consistant à entamer la face interne, on dilate radialement la zone à entamer ;
- on met les ouvertures en contact avec la composition en faisant circuler la composition dans des canaux dont une face est formée par la face interne du conduit ;
- on met les ouvertures en contact avec la composition en faisant circuler la composition dans des canaux dont une face est formée par une paroi présentant des orifices externes;
- le conduit est un vaisseau sanguin, par exemple, une artère ;
- le vaisseau présente une obstruction partielle ;
- le vaisseau porte un stent;
- la composition est destinée à la misé en oeuvre d'un traitement par thérapie génique.

On prévoit enfin selon l'invention une méthode pour administrer une composition dans une paroi d'un conduit d'un corps humain ou animal, caractérisée en ce qu'elle comprend les étapes consistant à :
- introduire le dispositif de l'invention dans le conduit ;
- étendre radialement les organes de coupe ou de perforation pour entamer la face interne de la paroi en réalisant des ouvertures borgnes dans l'épaisseur de la paroi ;
- disposer les moyens de distribution ;
- étendre radialement les moyens de distribution ; et
- mettre la composition en contact avec les ouvertures.

L'invention concerne par ailleurs un dispositif pour administrer une composition dans une paroi d'un conduit d'un corps humain ou animal, le dispositif comprenant des moyens aptes à entamer une face interne de la paroi du conduit pour réaliser des ouvertures borgnes dans une épaisseur de la paroi et des moyens de distribution pour mettre la composition en contact avec les ouvertures.

Le dispositif pourra présenter de plus au moins l'une des caractéristiques suivantes :
- les moyens aptes à entamer comprennent des organes de coupe ou de perforation ;
- les moyens pour entamer sont extensibles radialement par référence à une direction axiale du dispositif ;
- les moyens pour entamer sont associés à une chambre gonflable;
- les organes pour entamer sont portés par une paroi de la chambre gonflable ;
- les moyens pour entamer sont associés à un tube sur lequel est monté une chambre gonflable ;
- les organes pour entamer sont des organes de coupe ou de perforation
- les organes de coupe ou de perforation sont portés par le tube sur lequel est monté la chambre gonflable ;
- les moyens pour entamer comprennent des bras portant les organes de coupe ou de perforation ;
- les bras entourent la chambre gonflable;
- les moyens de distribution sont extensibles radialement par référence à une direction axiale du dispositif;
- les moyens de distribution présentent des canaux aptes à recevoir la composition, les canaux étant ouverts en direction opposée à un axe du dispositif ;
- les moyens de distribution comprennent une paroi présentant des orifices externes ;
- les moyens de distribution sont aptes à entourer les moyens pour entamer;
- les moyens de distribution sont aptes à coulisser par rapport aux moyens pour entamer suivant une direction axiale du dispositif;
- la chambre gonflable est apte à étendre radialement les moyens de distribution;
- le dispositif est destiné à administrer une composition dans la paroi d'un vaisseau sanguin tel qu'une artère, notamment une artère portant un stent;
- il s'agit d'un cathéter.

On prévoit en outre selon l'invention un dispositif pour administrer une composition dans une paroi d'un conduit d'un corps humain ou animal, le dispositif comprenant des moyens aptes à entamer une face interne de la paroi du conduit pour réaliser des ouvertures borgnes dans l'épaisseur de la paroi, ces moyens portant des organes de coupe ou de perforation et étant extensibles radialement par référence à un axe du dispositif, le dispositif comportant des moyens de distribution pour mettre la composition en contact avec les ouvertures, les moyens de distribution étant extensibles radialement et aptes à entourer les moyens pour entamer. Les moyens pour entamer selon l'invention sont tels qu'ils permettent de réaliser des ouvertures borgnes dans une épaisseur de la paroi telles décrites précédemment.

La méthode et le dispositif de l'invention concernent l'administration *in vivo* de compositions, notamment de compositions pharmaceutiques.

Selon un premier mode de réalisation préféré, il s'agit de compositions destinées à la mise en oeuvre de traitement de thérapie génique. Dans ce cas, ladite composition renferme au moins une information génétique d'intérêt, préférentiellement associée à un vecteur de transfert qui est destiné à permettre ou faciliter le transfert de cette information vers ou/et dans les cellules ciblés. Une telle information génétique d'intérêt consiste, ou est comprise, en une séquence d'acide nucléique.

Par « acide nucléique » ou « séquence d'acide nucléique », on entend désigner un fragment d'ADN et/ou d'ARN, double brin ou simple brin, linéaire ou circulaire, naturel isolé ou de synthèse, désignant un enchaînement précis de nucléotides, modifiés ou non, permettant de définir un fragment ou une région d'un acide nucléique sans limitation de taille. Selon un mode de réalisation préféré, cet acide nucléique est choisi parmi le groupe consistant en un cADN ; un ADN génomique ; un ADN plasmidique ; un ARN messager ; un ARN antisens; un ribozyme; un ARN de transfert; un ARN ribosomique; ou un ADN codant pour de tels ARN. De manière tout à fait préférée, un tel acide nucléique code pour un polypeptide ; on parlera alors de gène.

Un « vecteur de transfert » selon l'invention est destiné à permettre ou à faciliter le transfert d'une dite information génétique ou/et d'undit acide nucléique vers ou/et dans les cellules cibles. Il peut par exemple s'agir d'un plasmide libre de tout composé facilitant son introduction dans les cellules mais comprenant une dite information génétique; un tel plasmide ou untel acide nucléique comprenant une dite information génétique associé à au moins un polypeptide, notamment un polypeptide d'origine virale, et plus particulièrement d'origine adénovirale ou rétrovirale, préférablement undit acide nucléique incorporé dans une particule virale infectieuse (dans un cas préféré, ledit acide nucléique consistera en un génome viral, éventuellement modifié comme cela est proposé plus loin et recombiné en ce sens qu'il renferme une dite information génétique d'intérêt), ou un polypeptide synthétique; un acide nucléique associé à un ligand.

De façon préférée, selon la présente invention, « vecteur de transfert » désigne un vecteur recombinant d'origine plasmidique ou virale. Le choix des plasmides utilisables dans le cadre de la présente invention est vaste. Il peut s'agir de vecteurs de clonage et/ou d'expression. D'une manière générale, ils sont connus de l'homme du métier et nombre d'entre eux sont disponibles commercialement, mais il est également possible de les construire ou les modifier par les techniques de manipulation génétique. On peut citer à titre d'exemples les plasmides dérivés de pBR322 (Gibco BRL), pUC (Gibco BRL), pBluescript (Stratagène), pREP4, pCEP4 (Invitrogene) ou encore p Poly (Lathe et al., 1987, Gene 57, 193-201). De préférence, un plasmide mis en oeuvre dans le cadre de la présente invention contient une origine de réplication assurant l'initiation de la réplication dans une cellule productrice et/ou une cellule hôte (par exemple, on retiendra l'origine ColE1 pour un plasmide destiné à être produit dans *E. coli* et le système oriP/EBNA1 si l'on désire qu'il soit autoréplicatif dans une cellule hôte mammifère, (Lupton et Levine, 1985, Mol. Cell. Biol. 5, 2533-2542 ; Yates et al., Nature 313, 812-815). Il peut en outre comprendre un gène de sélection permettant de sélectionner ou identifier les cellules transfectées (par exemple complémentation d'une mutation d'auxotrophie, gène codant pour la résistance à un antibiotique). Bien entendu, il peut comprendre des éléments supplémentaires améliorant son maintien et/ou sa stabilité dans une cellule donnée (séquence *cer* qui favorise le maintien sous forme de monomère d'un plasmide (Summers et Sherrat, 1984, Cell *36*, 1097-1103), séquences d'intégration dans le génome cellulaire .

S'agissant d'un vecteur viral, on peut envisager un vecteur dérivant d'un poxvirus (par exemple virus de la vaccine, notamment MVA, canaripox), d'un adénovirus, d'un rétrovirus, d'un virus de l'herpès, d'un alphavirus (par exemple virus de la famille des Togavirus, notamment Semliki Forest virus), d'un foamy virus ou d'un virus associé à l'adénovirus. On aura de préférence recours à un vecteur non réplicatif et non intégratif. A cet égard, les vecteurs adénoviraux conviennent tout particulièrement à la mise en oeuvre de la présente invention. Toutefois, il convient de noter ici que dans le cadre de la mise en oeuvre de la présente invention, la nature du vecteur revêt peu d'importance.

Les rétrovirus ont la propriété d'infecter et de s'intégrer majoritairement dans les cellules en division et à cet égard sont particulièrement appropriés pour l'application visant à agir sur le phénomène de la resténose. Un rétrovirus recombinant utilisable dans le cadre de l'invention comporte généralement les séquences LTR, une région d'encapsidation et la séquence nucléotidique selon l'invention placée sous le contrôle du LTR rétroviral ou d'un promoteur interne tels que ceux décrits ci-après. Il peut dériver d'un rétrovirus d'une origine quelconque (murin, primate, félin, humain, etc.) et en particulier du MoMuLV (Moloney murine leukemia virus), MVS (Murine sarcoma virus) ou Friend murine retrovirus (Fb29). Il est propagé dans une lignée d'encapsidation capable de fournir en *trans* les polypeptides viraux gag, pol et/ou env nécessaires à la constitution d'une particule virale. De telles lignées sont décrites dans la littérature (PA317, Psi CRIP GP + Am-12 etc...). Le vecteur rétroviral selon l'invention peut comporter des modifications notamment au niveau des LTR (remplacement de la région promotrice par un promoteur eucaryote) ou de la région d'encapsidation (remplacement par une région d'encapsidation hétérologue, par exemple de type VL30) (voir les demandes françaises FR 94 08300 et FR 97 05203).

On pourra également avoir recours à un vecteur adénoviral défectif pour la réplication, c'est à dire dépourvu de tout ou partie d'au moins une région essentielle à la réplication sélectionnée parmi les régions E1, E2, E4 et/ou L1-L5. Une délétion de la région E1 est préférée. Mais elle peut être combinée à d'autres modification(s) / délétion(s) touchant notamment tout ou partie des régions E2, E4 et/ou L1-L5, dans la mesure où les fonctions essentielles défectives sont complémentées *en trans* au moyen d'une lignée de complémentation et/ou d'un virus auxiliaire afin d'assurer la production des particules virales d'intérêt. A cet égard, on peut avoir recours aux vecteurs de l'art antérieur tels que par exemple ceux décrits dans les demandes internationales WO 94/28152 et WO 97/04119. A titre illustratif, la délétion de la majorité de la région E1 et de l'unité de transcription E4 est tout particulièrement avantageuse. Dans le but d'augmenter les capacités de clonage, le vecteur adénoviral peut en outre être dépourvu de tout ou partie de la région E3 non essentielle. Selon une autre alternative, on peut mettre en oeuvre un vecteur adénoviral minimal retenant seulement les séquences essentielles à l'encapsidation, à savoir les ITRs (Inverted Terminal Repeat) 5' et 3' et la région d'encapsidation. Par ailleurs, l'origine du vecteur adénoviral selon l'invention, peut être variée aussi bien du point de vue de l'espèce que du sérotype. Il peut dériver du génome d'un adénovirus d'origine humaine ou animale (par exemple canine, aviaire, bovine, murine, ovine, porcine, simienne) ou encore d'un hybride comprenant des fragments de génome adénoviral d'au moins deux origines différentes. On peut citer plus particulièrement les adénovirus CAV-1 ou CAV-2 d'origine canine, DAV d'origine aviaire ou encore Bad de type 3 d'origine bovine (Zakharchuk et al., Arch. Virol., 1993, 128: 171-176 ; Spibey et Cavanagh, J. Gen. Virol., 1989, 70: 165-172 ; Jouvenne et al., Gene, 1987, 60: 21-28 ; Mittal et al., J. Gen. Virol., 1995, 76: 93-102). Cependant, on préférera un vecteur adénoviral d'origine humaine dérivant de préférence d'un adénovirus de sérotype C, notamment de type 2 ou 5. Un vecteur adénoviral selon la présente invention peut être généré in vitro dans Escherichia coli (E. coli) par ligation ou recombinaison homologue (voir par exemple WO 96/17070) ou encore par recombinaison dans une lignée de complémentation. Les différents vecteurs adénoviraux ainsi que leurs techniques de préparation sont connus (voir par exemple Graham et Prevect, 1991, in Methods in Molecular Biology, vol 7, p 109-128 ; Ed : E.J. Murey, The Human Press Inc).

On pourra également avoir recours à un vecteur viral réplicatif ou conditionnellement défectif pour la réplication. De tels vecteurs sont bien connus de l'homme de l'art et largement décrits dans la littérature.

S'agissant d'un vecteur non viral, il concernera plus spécifiquement le cas selon lequel un vecteur plasmidique tel que présenté ci-dessus est associé à un composé ou une combinaison de plusieurs composés permettant de faciliter son transfert à l'intérieur des cellules. De tels composés permettent en particulier d'améliorer l'efficacité transfectionnelle et/ou la stabilité d'un vecteur, particulièrement d'un vecteur d'origine plasmidique, et/ou la protection dudit vecteur *in vivo* à l'égard du système immunitaire de l'organisme hôte (Rolland A, Critical reviews in Therapeutic Drug Carrier System, 15, (1998), 143-198). Ces substances s'associent aux acides nucléiques par interaction électrostatique, hydrophobe, cationique, covalente ou préférentiellement non covalente. De telles substances sont largement documentées dans la littérature accessible à l'homme du métier (voir par exemple Felgner et al., 1987, Proc. West. Pharmacol. Soc. 32, 115-121 ; Hodgson et Solaiman , 1996, Nature Biotechnology 14, 339-342 ; Remy et al., 1994, Bioconjugate Chemistry 5, 647-654). A titre illustratif, mais non limitatif, il peut s'agir de polymères cationiques, de lipides cationiques, mais également de liposomes, de protéines nucléaires ou virales ou encore de lipides neutres. Ces substances peuvent être utilisées seules ou en combinaison. Des exemples de tels composés, ainsi que de méthodes permettant de mesurer leur capacité à améliorer l'efficacité transfectionnelle et/ou la stabilité d'un vecteur donné, sont notamment disponibles dans les demandes de brevet WO 98/08489, WO 98/17693, WO 98/34910, WO 98/37916, WO 98/53853, EP 890362 ou WO 99/05183. Il peut notamment s'agir de substances lipidiques telles que le DOTMA (Felgner et al., 1987, PNAS, 84, 7413-7417), le DOGS ou Transfectam™ (Behr et al., 1989, PNAS, 86, 6982-6986), de la DMRIE ou DORIE (Felgner et al., 1993, Methods, 5, 67-75), du DC-CHOL (Gao et Huang, 1991, BBRC, 179, 280-285), du DOTAP™ (McLachlan et al., 1995, Gene Therapy, 2, 674-622) ou de la Lipofectamine™. Il peut également s'agir d'un polymère cationique tel que par exemple la polyamidoamine (Haensler et Szoka, Bioconjugate Chem. 4 (1993), 372-379), un polymère "dendrimer" (WO 95/24221), du polyéthylène imine ou du polypropylène imine (WO 96/02655), du chitosan, un poly(aminoacide) comme la polylysine (US- 5,595,897 or FR-2 719 316) ; un composé polyquaternaire ; la protamine; les polyimines; le polyéthylène imine ou le polypropylène imine (WO 96/02655); les polyvinylamines; les polymères polycationiques substitués par le DEAE, comme les pullulanes, les celluloses; la polyvinylpyridine; les polymethacrylates; les polyacrylates; les polyoxéthanes; le polythiodiethylaminomethylethylene (P(TDAE)); la polyhistidine; la polyornithine; le poly-p-aminostyrène; les polyoxéthanes; les co-polymethacrylates (par exemple les copolymères d'HPMA; N-(2-hydroxypropyl)-methacrylamide); les composés décrits dans US-A-3,910,862, les complexes de polyvinylpyrrolide de la DEAE avec le méthacrylate, le dextran, l'acrylamide, les polyimines, l'albumine, le 1-dimethylaminomethylmethacrylate et le chlorure d'ammonium de polyvinylpyrrolidonemethylacrylaminopropyltrimethyl; les polyamidoamines; les composés télomériques (demande de brevet EP 98401471.2). Néanmoins, cette liste n'est pas exhaustive et d'autres polymères cationiques connus peuvent être utilisés pour obtenir les complexes d'acides nucléiques de l'invention. De plus ces lipides et polymères cationiques peuvent être fluorinés (voir par exemple WO 98/34910). Dans un cas avantageux, de tels vecteurs non-viraux renferment en outre un adjuvant tel que par exemple un lipide neutre, zwitterionique ou chargé négativement. Ces lipides neutres, zwitterioniques ou chargés négativement peuvent être, par exemple, sélectionnés dans le groupe comprenant les phospholipides naturels d'origine animale ou végétale, tels que la phosphatidylcholine, la phosphocholine, la phosphatidyléthanolamine, la sphingomyéline, la phosphatidylsérine, le phosphatidylinositol , la céramide ou la cérebroside et leurs analogues ; les phospholipides synthétiques qui comportent généralement, mais non exclusivement deux chaînes d'acide gras identiques, tels que la dimyristoylphosphatidylcholine, la dioleoylphosphatidylcholine, la dipalmitoylphosphatidylcholine, la distearoylphosphatidylcholine, la phosphatidyléthanolamine (PE) et le phosphatidylglycérol, et leurs analogues ; la phosphatidylcholine, la cardiolipine, la phosphatidyléthanolamine, mono-, di- ou triacylglycérol, et l'alpha-tocophérol et leurs analogues ; le phosphatidylglycérol, l'acide phosphatidique ou l'analogue d'un phospholipide similaire ; le cholestérol, les glycolipides, les acides gras, les sphingolipides, les prostaglandines, les gangliosides, les niosomes, ou tout autre amphiphile naturel ou synthétique.

Selon un cas préféré, ladite information génétique d'intérêt comprend ou consiste en un «acide nucléique renfermant une séquence codant pour un polypeptide d'intérêt» on entend ainsi indiquer que ledit acide nucléique comprend un gène codant pour un polypeptide d'intérêt, et des éléments d'expression d'undit gène. Le terme « polypeptide » s'entend sans restriction quant à sa taille ou son degré de glycosylation.

Dans le cas où l'acide nucléique comprend une séquence codant pour un polypeptide d'intérêt, il convient de préciser que ledit acide nucléique comporte en outre les éléments nécessaires afin d'assurer l'expression de ladite séquence après transfert dans une cellule cible, notamment des séquences promotrices et/ou des séquences de régulation efficaces dans ladite cellule, et éventuellement les séquences requises pour permettre l'excrétion ou l'expression à la surface des cellules cibles dudit polypeptide. Les éléments nécessaires à l'expression sont constitués par l'ensemble des éléments permettant la transcription de la séquence nucléotidique en ARN et la traduction de l'ARNm en polypeptide, notamment les séquences promotrices et/ou des séquences de régulation efficaces dans ladite cellule, et éventuellement les séquences requises pour permettre l'excrétion ou l'expression à la surface des cellules cibles dudit polypeptide. Ces éléments peuvent être régulables ou constitutifs. Bien entendu, le promoteur est adapté au vecteur retenu et à la cellule hôte. On peut citer, à titre d'exemples, les promoteurs eucaryotes des gènes PGK (Phospho Glycérate Kinase), MT (metallothioneine ; Mc Ivor et al., 1987, Mol. Cell Biol., 7, 838-848), α-1 antitrypsine, CFTR, les promoteurs du gène codant pour la créatine kinase musculaire, pour l'actine, pour les immunoglobulines, pour la β-actine (Tabin et al., 1982, Mol. Cell Biol., 2, 426-436), SRα (Takebe et al., 1988, Mol. Cell. Biol., 8, 466-472), le promoteur précoce du virus SV40 (Simian Virus), le LTR du RSV (Rous Sarcoma Virus), le promoteur de MPSV, le promoteur TK-HSV-1, le promoteur précoce du virus CMV (Cytomegalovirus), les promoteurs du virus de la vaccine p7.5K pH5R, pK1L, p28, p11 et les promoteurs adénoviraux E1A et MLP ou une combinaison desdits promoteurs. Le promoteur précoce du Cytomégalovirus (CMV) est tout particulièrement préféré. Il peut également s'agir d'un promoteur stimulant l'expression du gène spécifiquement dans une cellule musculaire lisse. On peut citer notamment les promoteurs des gènes de l'α-actine des muscles lisses (Foster et al., 1992, J. Biol. Chem. 267,11995-12003 ; Shimizu et al., 1995, J. Biol. Chem. 270, 7631-7643), de la chaine lourde de myosine de muscle lisse (Katoh et al.,1994,J. Biol. Chem. 269,30538-30545), de la desmine (EP0 999 278 ; Mericskay et al., 1999, Current Topics in Pathology Vol 93 p7-17 Eds Desmoulière et Tuchweber, Springer-Verlag Berlin Heidelberg), du SM22α (Kim et al., 1997, J. Clin. Invest. 100, 1006-14). S'agissant de promoteurs spécifiques on peut plus particulièrement envisager des promoteurs chimériques permettant une expression dans les cellules musculaires lisses à la fois forte et spécifique. Par exemple un promoteur tel que décrit dans le document de priorité EP 00 44 0208.7 concernant une construction chimère comprenant un enhancer muscle spécifique et un promoteur spécifique des SMC ( smooth muscle cell) en particulier sélectionné parmi ceux des gènes de SM α-actin, SM chaîne lourde de myosine (SM-MHC), desmine ou SM22α. Il est également possible d'utiliser une région promotrice tissu-spécifique et/ou activable dans des conditions définies. La littérature procure un grand nombre d'informations relatives à de telles séquences promotrices. Par ailleurs, ledit acide nucléique peut renfermer au moins deux séquences, identiques ou différentes, présentant une activité de promoteur transcriptionnel et/ou au moins deux séquences codant pour un polypeptide d'intérêt, identiques ou différentes, situées l'une par rapport à l'autre de manière contiguë, éloignée, dans le même sens ou en sens inverse, pour autant que la fonction de promoteur transcriptionnel ou la transcription desdites séquences ne soit pas affectée. Dans le cas où l'acide nucléique renferme au moins deux séquences codant pour un polypeptide. De même, dans ce type de construction d'acide nucléique, il est possible d'introduire des séquences nucléiques « neutres » ou introns qui ne nuisent pas à la transcription et sont épissées avant l'étape de traduction. De telles séquences et leurs utilisations sont décrites dans la littérature (WO 94/29471). Ledit acide nucléique pourra également renfermer des séquences requises pour le transport intracellulaire, pour la réplication et/ou pour l'intégration, pour la sécrétion, pour la transcription ou la traduction. De telles séquences sont bien connues de l'homme du métier. Par ailleurs, les acides nucléiques utilisables selon la présente invention peuvent également être des acides nucléiques modifiés de sorte qu'il ne leur est pas possible de s'intégrer dans le génome de la cellule cible ou des acides nucléiques stabilisés à l'aide d'agents, tels que par exemple la spermine, qui en tant que tels n'ont pas d'effet sur l'efficacité de la transfection.

Dans le cadre de la présente invention, il est possible d'utiliser l'intégralité ou une partie seulement de la séquence d'acide nucléique codant pour le polypeptide d'intérêt, ou un polypeptide dérivé ou muté, dans la mesure où la fonction et les propriétés recherchées desdits polypeptides sont maintenues. Au sens de la présente invention, on entend par mutation, une délétion et / ou une substitution et / ou une addition d'un ou plusieurs nucléotides. De même, il est envisageable d'utiliser une séquence codant pour un polypeptide hybride provenant de la fusion de la séquence codant pour un polypeptide d'intérêt selon l'invention et de la séquence codant pour un polypeptide d'un autre type (par exemple, cytotoxique, d'ancrage membranaire, de sécrétion).

Par « information génétique d'intérêt ou séquence d'acide nucléique codant pour un polypeptide d'intérêt ou gène » et dans le cadre d'une application selon l'invention, tout particulièrement relative au traitement ou à la prévention de laresténose et/ou de la re-resténose, on entend désigner par exemple les gènes codant pour des inhibiteurs de la migration et de la prolifération des cellules musculaires lisses de la paroi artérielle , les gènes codant pour un polypeptide présentant une activité vasoprotectrice, cytostatique, proapoptotique ou cytotoxique. Des exemples sont proposés ci-dessous ou dans les documents suivants dont les contenus font partie intégrante de la demande par référence , par exemple on pourra citer, Kibbe et al., 2000, Circ. Res. 86, 829-33 ; Macejak et al., 1999, J. Virol. 73, 7745-51 ; Claudio et al., 1999, Circ. Res. 85, 1032-9 ; Perlman et al., 1999, Gene Ther. 6, 758-63.

Des exemples de polypeptides codés par le gène d'intérêt selon la présente invention incluent, sans pour autant se limiter à ces exemples :
- des polypeptides impliqués dans le cycle cellulaire tel que p21,p16, le produit d'expression du gène du rétinoblastome (Ab), des inhibiteurs de kinase, de préférence du type dépendant de cycline GAX, GAS-1, GAS-3, GAS-6, GADD-45, GADD-153 et cycline A, B et D, des inhibiteurs de c-myc, c-myb, Cdk et H-ras
- des polypeptides impliqués dans l'apoptose, comme p53, Bas, Bc12, Bcl1X, Bad ou d'autres antagonistes,
- des polypeptides angiogéniques, tels que les membres de la famille des facteurs de croissance endothéliaux (VEGF), des facteurs de croissance transformant (TGF et particulièrement TGFα et β), des facteurs de croissance épithéliaux (EGF), des facteurs de croissance des fibroblastes (FGF, et particulièrement FGFa et FGFb), des facteurs de nécroses des tumeurs (TNF et particulièrement TNFα et TNFβ), des protéines de la famille CCN (qui inclut CTGF, Cyr61, Nov, Elm-1, Cop-1 et Wisp-3), les facteurs de dispersion/facteurs de croissance d'hépatocyte (SH/HGF), l'angiogénine, l'angiopoïétine (en particulier 1 et 2), l'angiotensine-2, cytokines (qui incluent en particuliers les interférons β et γ),
- des polypeptides capables de diminuer ou d'inhiber une prolifération cellulaire, qui incluent des anticorps, des toxines, des immunotoxines, des polypeptides inhibiteurs, les produits d'expression d'oncogène (ras, MAP kinase, les récepteurs tyrosine kinase, les facteurs de croissance), le ligand de fas, des produits de gène suicide (par exemple, HSV-tk, cytosine désaminase),
- des polypeptides capables de diminuer ou d'inhiber une migration cellulaire,
- des polypeptides capables de moduler ou de réguler l'expression de gènes cellulaires,
- des facteurs de coagulation (Facteur VIII, Facteur IX, ...),
- des enzymes tels que l'uréase, la rénine, la thrombine, les métalloprotéinases, les synthases de monoxides d'azote (eNOS ou iNOS), SOD, Catalase, oxygénase d'hème, la famille des lipases lipoprotéines,
- les peptides natriurétiques A, B et C
- des récupérateurs de radicaux oxydés,
- des inhibiteurs d'enzymes, tels que l'alpha1-antitrypsine, l'antithrombine III, l'inhibiteur de l'activateur du plasminogène PAI-1, l'inhibiteur tissulaire des metalloprotéinases (TIMP 1-4) ,
- des facteurs de transcription, tels que les récepteurs nucléaires qui comprennent un domaine de liaison de l'ADN, un domaine de liaison d'un ligand, et un domaine d'activation ou d'inhibition de la transcription (par exemple, des produits fusions dérivés des récepteurs à l'oestrogène, aux stéroides
ou à la progestérone,
- des marqueurs (β-galactosidase, CAT, luciférase, GFP...)
- et tout polypeptide qui est reconnu dans l'art comme étant utile pour le traitement ou la prévention d'une condition clinique, et particulièrement ceux pour lesquels il est souhaitable d'obtenir une expression dans les cellules présentes dans les parois de conduits humains ou animaux, tel que les parois des vaisseaux.

Le polypeptide d'intérêt qui est codé par la séquence comprise dans ledit acide nucléique est choisi de préférence parmi les polypeptides présentant une activité antiproliférative ou anti-migratoire, les facteurs protéiques vaso-protecteurs, les facteurs protéiques angiogéniques et les polypeptides présentant une activité d'activation de l'apoptose cellulaire, les cytokines, les protéines codées par un gène appelé « gène suicide ». Les cytokines sont des molécules naturellement produites à la suite d'une stimulation antigénique ou d'une réaction inflammatoire (Gillis and Williams, 1998, Curr. Opin. Immunol., 10, 501-503) dont l'utilité dans le cadre du traitement de la resténose a été montrée notamment par Stéphan D (Mol Med, 1997, 3, 593-9). Selon cette variante de l'invention, le polypeptide d'intérêt désignera préférentiellement les interférons β et γ qui sont capables d'inhiber la prolifération des cellules musculaires lisses *in vitro* et *in vivo* (Stopeck A, 1997, Cell transplantation, 6, 1-8).

Selon l'invention, le polypeptide d'intérêt peut également être un polypeptide présentant une activité anti-migratoire. Selon cette variante, le polypeptide d'intérêt désignera préférentiellement un inhibiteur de metalloprotéinases (TIMP1-4) capable d'inhiber la digestion de la matrice extracellulaire et donc de diminuer la migration des cellules musculaires lisses de la média vers l'intima (Cheng L, 1998, Circulation, 98, 2195-2201).

Selon une autre variante de l'invention, le polypeptide d'intérêt est un polypeptide présentant une activité vaso-protectrice. Selon cette variante de l'invention, le polypeptide d'intérêt est préférentiellement un vasorelaxant capable de réguler la prolifération des cellules musculaires lisses et d'exercer une action vaso-protectrice en induisant une accumulation de cGMP (Hikaru U, 1997, Circulation, 96, 2272-2279).

Selon une autre variante de l'invention, le polypeptide d'intérêt est un polypeptide présentant une activité angiogénique. Les rôles potentiels du facteur plaquétaire (PDGF), de la thrombospondine, des facteurs fibroblastiques (FGFs), des facteurs de croissance transformant (TGF et particulièrement TGFα et β) et des facteurs de croissance épithéliaux (EGF) sur la prévention de la resténose ont été discutés (Cerek, 1991, Am. J. Cardiol., 68, 24-33) et le rôle du facteur de croissance endothélial (VEGF) a plus particulièrement été mis en évidence in vivo par son action sur la réendothélialisation de l'artère lésée (Asahara, Circulation, 1994, 3291-3302)

Selon une autre variante de l'invention, le polypeptide d'intérêt est un polypeptide codé par un gène appelé « gène suicide ». De nombreux couples gène suicide/prodrogue sont actuellement disponibles. On peut citer plus particulièrement, les couples (a) thymidine kinase du virus herpès simplex de type 1 (TK HSV-1) et acyclovir ou ganciclovir (GCV) et (b) cytosine désaminase (CDase) et 5-fluorocytosine (5FC) ayant démontré une capacité à inhiber la prolifération néointimale en modèle animal (Takeshi O, 1994, Science, 781-784 ; Harrell R, 1997, Circulation, 96, 621-627 et les couples purine nucleoside phosphorylase d'Escherichia coli (E. Coli) et 6-methylpurine deoxyribonucleoside (Sorscher et al., 1994, Gene Therapy 1, 233-238) ; guanine phosphoribosyl transférase d'E. coli et 6- thioxanthine (Mzoz et Moolten, 1993, Human Gene Therapy 4, 589-595).

Selon un cas avantageux , l'invention concerne le cas selon lequel ledit polypeptide d'intérêt présente au moins une activité enzymatique sélectionnée parmi l'activité thymidine kinase, l'activité purine nucléoside phosphorylase, l'activité guanine ou uracile ou orotate phosphoribosyl transférase et l'activité cytosine désaminase.

Enfin, le polypeptide d'intérêt peut être un polypeptide présentant une activité d'activation de l'apoptose cellulaire, et plus particulièrement le ligand de Fas qui est capable d'inhiber la formation de néointima (Luo Z, 1999, Circulation, 99, 1776-1779).

Les séquences codant pour les polypeptides d'intérêt de l'invention peuvent être aisément obtenues par clonage, par PCR ou par synthèse chimique selon les techniques conventionnelles en usage. Il peut s'agir de gènes natifs ou dérivés de ces derniers par mutation, délétion, substitution et/ou addition d'un ou plusieurs nucléotides. Par ailleurs, leurs séquences sont largement décrites dans la littérature consultable par l'homme du métier.

Avantageusement, la composition destinée à être administrée, en fonction de la nature du vecteur utilisé, comprendra :
- lorsque le vecteur est d'origine plasmidique, ou vecteur non viral, de 0,01 à 100 mg d'ADN, de préférence entre 0,05 à 10 mg et, de manière tout à fait préférée de 0,5 à 5 mg ;
- lorsque le vecteur est d'origine virale, entre 10⁴ et 10¹⁴ ufp (unités formant des plages), avantageusement entre 10⁵ et 10¹³ ufp et de préférence entre 10⁶ et 10¹² ufp.

Ces dosages sont fournis à titre indicatifs étant entendu que le praticien pourra les adapter selon les besoins, l'état du patient, l'affection à traiter ou à prévenir, le gène, le vecteur, le promoteur utilisés, etc... sans que cela réclame un travail excessif de mise au point. En outre, de tels ajustements sont totalement indépendants du dispositif selon l'invention ou de sa mise en oeuvre *in vivo*.

Selon un autre mode de réalisation, la composition administrée selon l'invention est une composition renfermant un composé actif, autre qu'un vecteur de transfert ou une information génétique ou acide nucléique tels que définis précédemment, que l'on souhaitera administrer dans un conduit humain ou animal, et plus particulièrement au niveau de ses parois. Selon l'invention, par «composé actif » on entend désigner un ou plusieurs agents biologiquement actifs, tels que par exemple des agents anti-inflammatoires qui préviennent l'inflammation, des composés prévenant la resténose en limitant la prolifération tissulaire, des composés anti-thrombogéniques qui inhibent ou contrôlent la formation de thrombus or la thrombolyse, ou des composés bioactifs qui régulent la croissance tissulaire et stimule la cicatrisation des tissus. De tels composés actifs sont par exemple, mais ne sont pas limités à, des stéroïdes, la fibronectine, des composés anti-coagulants, anti-plaquettaire, des composés empêchant la croissance des cellules musculaire lisses de la surface interne de la paroi des vaisseaux, l'héparine ou des fragments d'héparine, l'aspirine, la coumadine, l'activateur du plasminogène tissulaire (ou TPA), l'urokinase, l'hirudine, la streptokinase, les anti-prolifératifs (le méthotrexate, le cisplatine, le fluorouracile, l'adriamycine), les antioxydants (l'acide ascorbique, le béta carotène, la vitamine E), les anti-métabolites, les inhibituers de thromboxane, des anti-inflammatoires non-stéroïdiens et stéroïdiens, des bloquants de la pompe calcique, des immunoglobulines, des anticorps, des cytokines, lymphokines, facteurs de croissance, prostaglandines, leukotriènes, laminine, élastine, collagène, ou intégrines. Selon un cas particulier, de tels composés sont, préalablement à leur administration à l'aide du dispositif selon l'invention, encapsidés par exemple dans des liposomes, des nanoparticules ou des pharmacosomes. De tels procédés d'encapsulation ont été largement décrits dans la littérature et l'on se réfèrera par exemple aux documents US 5,874,111, US 5,827,531, US 5,773,027 ou US 5,770,222 dont les contenus sont incorporés ici par référence.

Les compositions qui pourront être administrées à l'aide du dispositif de l'invention peuvent en outre être formulées avec un véhicule acceptable d'un point de vue pharmaceutique. Un tel support est préférentiellement isotonique, hypotonique ou faiblement hypertonique et présente une force ionique relativement faible, tel que par exemple une solution de sucrose. Par ailleurs, un tel support peut renfermer tout solvant, ou liquide aqueux ou partiellement aqueux tel que de l'eau stérile non pyrogène. Le pH de la formulation est en outre ajusté et tamponné afin de répondre aux exigences d'utilisation *in vivo*. La formulation peut également inclure un diluant, un adjuvant ou un excipient acceptable d'un point de vue pharmaceutique, de même que des agents de solubilisation, de stabilisation, de préservation. Pour une administration injectable, on préfère une formulation en solution aqueuse, non-aqueuse ou isotonique. Elle peut être présentée en dose unique ou en multidoses, sous forme liquide ou sèche (poudre, lyophilisat...) susceptible d'être reconstituée de manière extemporanée par un diluant approprié. Selon un mode particulier de l'invention, cette composition pourra comporter en outre des quantités acceptables d'un point de vue pharmaceutique d'une prodrogue capable d'être transformée en molécule cytotoxique par un polypeptide ayant au moins une activité cytotoxique. Une telle prodrogue sera notamment sélectionnée dans le groupe consistant en l'acyclovir ou le ganciclovir (GCV), la cyclophosphophamide, la 6-methylpurine deoxyribonucleoside, la 6-thioxanthine, la cytosine ou un de ses dérivés ou l'uracile ou un de ses dérivés. De plus, lorsque ladite prodrogue est la 5-fluorocytosine (5FC) ou la 5-fluorouracile (5-FU ), ledit produit de combinaison peut également comprendre une ou plusieurs substances potentialisant l'effet cytotoxique du 5-FU. On peut citer en particulier, les drogues inhibant les enzymes de la voie de biosynthèse de novo des pyrimidines (par exemple celles citées ci-après), les drogues telles que la Leucovorin (Waxman et al., 1982, Eur. J. Cancer Clin. Oncol. 18, 685-692) qui en présence du produit du métabolisme du 5-FU (5-FdUMP) augmente l'inhibition de la thymidylate synthase ce qui entraîne une diminution du pool de dTMP nécessaire à la réplication et enfin les drogues telles que le méthotréxate (Cadman et al., 1979, Science 250, 1135-1137) qui en inhibant la dihydrofolate réductase et en élevant le pool d'incorporation de PRPP (phosphoribosylpyrophosphate) provoque l'augmentation de 5-FU dans l'ARN cellulaire.

De même la composition à administrer peut en outre renfermer une substance sélectionnée dans le groupe comprenant par exemple la chloroquine, les composées protiques comme le propylène glycol, le polyéthylène glycol, le glycérol, l'éthanol, le 1-méthyl L-2-pyrrolidone et les dérivés de ceux-ci, des composés aprotiques, comme par exemple le diméthylsulfoxide (DMSO), diéthylsulfoxide, di-n-propylsulfoxide, diméthylsulfone, sulfolane, diméthylformamide, diméthylacétamide, tetraméthylurée, acétonitrile ou leurs dérivés (voir EP 890 362), les cytokines, particulièrement l'interleukine-10 (IL-10) (WO 9956784), la hyaluronidase (WO 98/53853) et les inhibiteurs des nucléases (WO 9956784) comme par exemple l'actine G. Dans un autre mode de réalisation de l'invention cette substance peut-être un sel et de préférence un sel cationique comme par exemple le magnésium (Mg²⁺) (EP 998945) et/ou le lithium (Li⁺). Dans ce cas, la quantité de substance ionique dans le complexe d'acides nucléiques de l'invention varie avantageusement entre 0.1 mM et environ 100 mM, et préférentiellement entre 0.1mM et environ 10 mM.

Bien entendu, on pourra apporter à l'invention de nombreuses modifications sans sortir du cadre de celle-ci.

On pourra par exemple utiliser un dispositif selon l'invention comprenant un cathéter unique ou deux cathéters totalement séparés, l'un pour entamer la paroi, l'autre pour administrer la composition, bien que cela soit cliniquement moins avantageux.

On pourra également appliquer l'invention à d'autres conduits que les vaisseaux sanguins, par exemple, l'invention pourra être applicable à l'urologie et la gastroentérologie.

Enfin, les moyens pour entamer la paroi pourraient ne pas être seulement mécaniques. Ils pourront par exemple, mettre en oeuvre des sources laser, des moyens chimiques ou enzymatiques. Plus particulièrement on pourra utiliser des enzymes capables de digérer la matrice extracellulaire tels que la collagénase ou la hyaluronidase. L'hydrolyse du collagène et de l'acide hyaluronique par ces enzymes engendre une désorganisation de la matrice extracellulaire et facilite l'accès de la composition aux cellules cibles.

D'autres caractéristiques et avantages de l'invention apparaîtront encore dans la description suivante de trois modes préférés de réalisation de l'invention donnés à titre d'exemples non limitatifs.

### Exemple 1 :

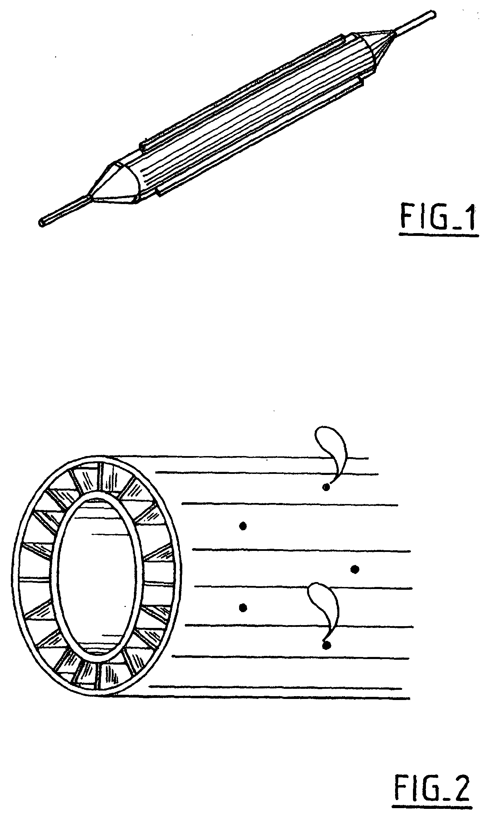

Les figures 1 et 2 montrent deux cathéters commerciaux dont l'association permet de mettre en oeuvre la méthode de l'invention.

Dans une première étape le cathéter « cutting balloon™ » (figure 1, Interventional Technologies, US 5,797,935) est avancé dans l'artère au site obstrué par la resténose intra-stent. La chambre gonflable est alors dilatée de façon à écraser la resténose et rétablir un diamètre acceptable de l'artère. Le cathéter « cutting balloon™ » possède à la surface de la chambre gonflable trois ou quatre lames de rasoirs microchirurgicales. Ces lames sont conçues pour rendre la dilatation de l'artère moins traumatique en pratiquant des microfractures dans la paroi et en diminuant les forces exercées sur l'artère. Lorsque la chambre gonflable est dilatée les lames de rasoirs se déploient radialement et créent des incisions dans le tissu resténotique.

Le cathéter « cutting balloon™ » est alors retiré et remplacé par le cathéter « Remedy balloon™ » (figure 2, Boston Scientific/SCIMED, US 5,792,105) qui est introduit au site dilaté de l'artère. La chambre gonflable de ce cathéter est dilatée et applique contre les ouvertures borgnes pratiquées dans la paroi de l'artère par le « cutting balloon™ » des canaux dont une face contient une paroi présentant des orifices externes. La composition est alors distribuée par les canaux et mise en contact avec les cellules de la paroi de l'artère par l'intermédaire des orifices.

Les deux cathéters utilisés sont donnés à titre d'exemples non limitatifs. En particulier les cathéters « expandable and compressible atherectomy catheter » (US 5,556,408), « universal dilator with reciprocal incisor » (US 5,556,405), « Angioplasty balloon with light incisor » (US 5,624,433), «improved vascular incisor/dilator » (US 5,649,941), « universal dilator with expandable incisor » (US 5,697,944), « device and method for transecting a coronary artery » (US 5,713,913) pourront être utilisés en remplacement de « cutting balloon™ ». Les cathéters « infiltrator® » (Interventional Technologies), « Crescendo™ » (Cordis), « InfusaSleeve™ » (LocalMed), « Dispatch catheter™ » (Boston Scientific/SCIMED), « Hydrogel-coated balloon catheter™ » (Boston Scientific/SCIMED) pourront être utilisés en remplacement de « Remedy balloon™ ».

### Exemple 2 :

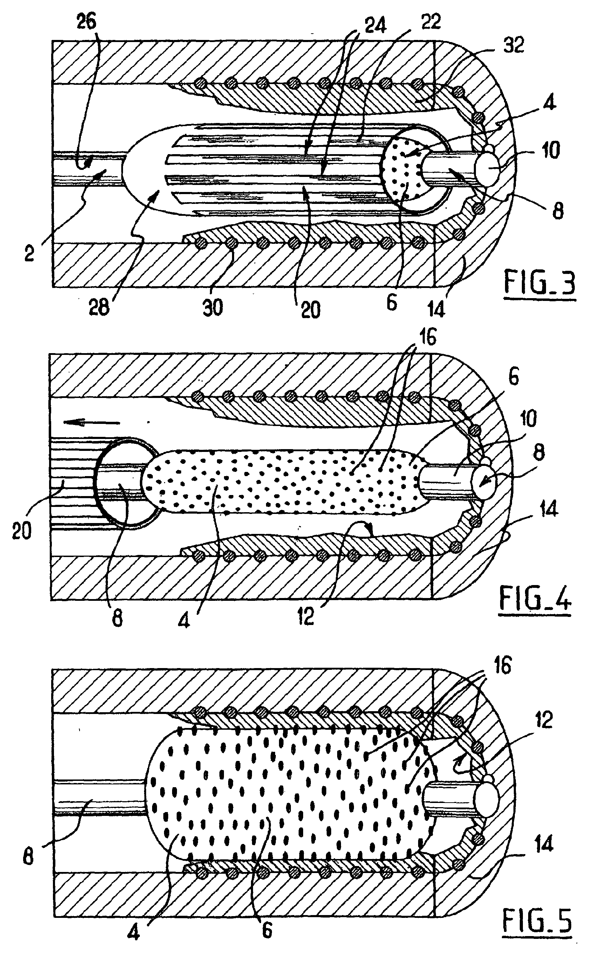

- les figures 3 à 10 montrent différentes étapes de mise en oeuvre de la méthode selon l'invention au moyen d'un premier mode de réalisation du cathéter selon l'invention ;

Le cathéter 2 présente une extrémité distale destinée à être introduite dans le conduit à traiter. Cette extrémité comporte un outil interne 4 comprenant un ballon 6 de forme générale cylindrique allongée, arrondie à ses deux extrémités axiales. Le ballon 6 est monté sur un tube 8 le traversant de part en part suivant son axe. Une extrémité distale 10 du tube émerge de l'extrémité distale du ballon. De façon connue en soi pour les cathéters, le tube 8 est creux et est en communication de fluide avec l'intérieur du ballon. De la sorte, le ballon peut être gonflé par amenée de liquide à travers le tube, à partir de l'extrémité proximale du cathéter, non-illustrée. Le gonflement du ballon entraîne son extension radiale par référence à l'axe du cathéter .

Le ballon 6 porte sur sa paroi, en saillie de la face externe, des organes 16 aptes à entamer la face interne 12 d'une paroi d'un conduit du corps humain, tel qu'une artère 14. Les organes sont en l'espèce des organes de perforation conformés en pointe, par exemple constitués par des cristaux.

L'extrémité distale du cathéter comporte de plus un outil externe 20. Cet outil est qualifié d'« externe » car il est destiné à s'étendre autour de l'outil interne 4. Mais il est bien entendu destiné à s'étendre dans le conduit 14, tout comme l'autre outil. L'outil externe 20 comprend un manchon 22 ayant une paroi souple elle aussi de forme générale cylindrique allongée. Cette paroi 22 est creuse en son centre. Elle est ouverte à son extrémité distale et a une extrémité proximale fermée de forme arrondie.

La paroi 22 présente, ménagés dans son épaisseur, des canaux allongés rectilignes 24 s'étendant parallèlement à l'axe du cathéter. Ces canaux ont un profil transversal (dans un plan perpendiculaire à l'axe) en forme générale de « U », le fond du canal correspondant à la base du « U » s'étendant du côté de l'axe.

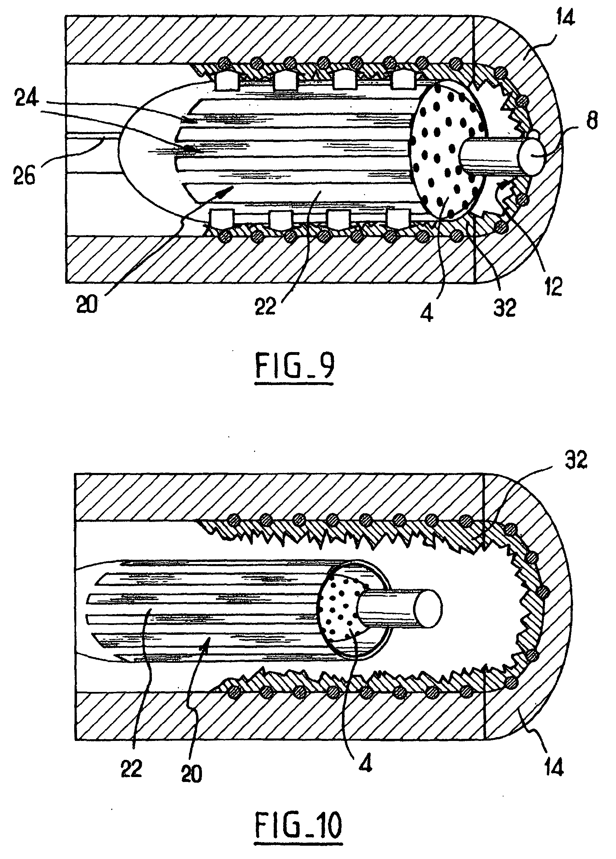

Comme on le voit notamment sur la figure 9, le manchon 22 est relié à un tube 26 par son extrémité proximale. Le tube 8 de l'outil interne est reçu à coulissement dans le tube 26 de l'outil externe.

Le manchon souple est extensible radialement de sorte qu'il peut accroître son diamètre.

Chaque canal 24 est en communication de fluide avec l'extrémité proximale du cathéter via une chambre de répartition 28 et via le tube 26 pour permettre l'amenée dans chaque canal d'une composition liquide à administrer à la paroi du vaisseau.

A l'extrémité proximale du cathéter de l'invention, celui-ci comprend des moyens d'actionnement et de commande de l'extrémité distale, ainsi que des moyens d'injection de fluides. Cette extrémité proximale s'étend à l'extérieur du corps du patient et est manipulée par le personnel effectuant l'intervention chirurgicale.

Le cathéter qui vient d'être décrit est employé de la façon suivante pour mettre en oeuvre la méthode selon l'invention.

On suppose que le conduit 14 à traiter est une artère coronaire humaine. Le tronçon à traiter présentait une plaque d'athérome qui a été traitée par expansion au moyen d'un cathéter à ballon d'un type classique, puis par la pose d'une stent maillé 30 d'un type connu en soi et dont la trace dans le plan de coupe est visible sur les figures 3 à 10. A la suite de la pose du stent, une cicatrisation excessive 32 du tronçon traité est intervenue, réduisant le diamètre interne de l'artère et réduisant la section disponible pour la circulation du sang. La méthode selon l'invention vise à limiter la reformation d'une cicatrisation excessive. Elle est destinée à traiter la resténose en prévenant la re-resténose.

En référence à la figure 3, on achemine dans l'artère en regard du tronçon à traiter l'extrémité distale 2 du cathéter. L'outil interne 4 avec le ballon dégonflé, s'étend dans l'outil externe 20, coaxialement à celui-ci.

En référence à la figure 4, une fois l'extrémité distale placée en regard du tronçon, on fait coulisser vers l'arrière l'outil externe 20 pour dégager l'outil interne 4.

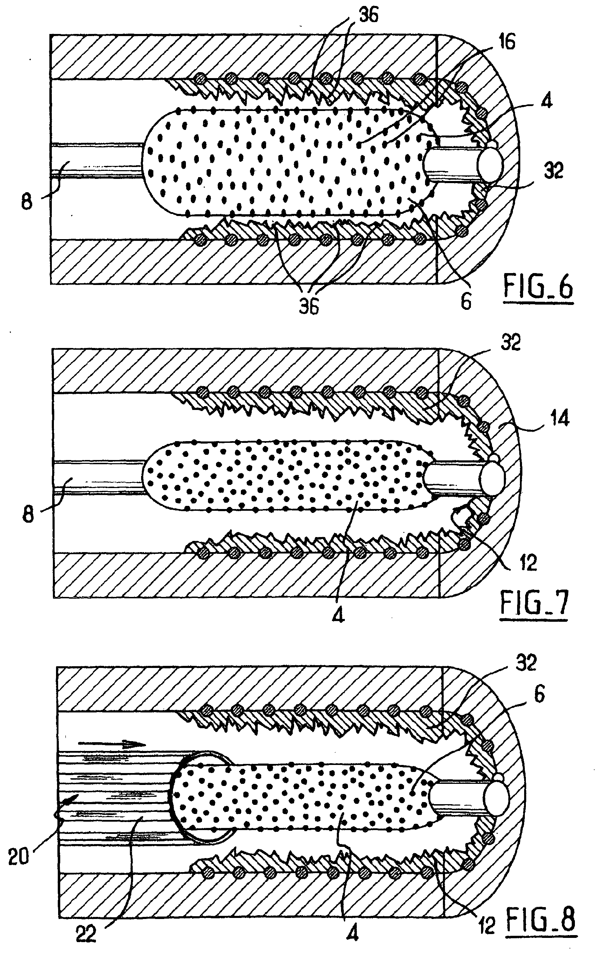

Comme l'illustre la figure 5, on gonfle ensuite le ballon 6 pour augmenter son diamètre de sorte que l'artère retrouve un diamètre interne acceptable et que les organes de perforation 16 pénètrent dans la face interne de l'artère. Ces organes réalisent des ouvertures borgnes radiales 36 dans l'épaisseur de la paroi de l'artère, à partir de sa face interne. Ces ouvertures s'étendent donc au coeur de cette paroi. Les ouvertures 36 ont été illustrées sur la figure 6. Elles sont bien sûr plus petites et en plus grand nombre que ce qui a été illustré.

En référence à la figure 7, on dégonfle ensuite le ballon 6 pour réduire son diamètre.

On fait alors à nouveau coulisser axialement vers l'avant l'outil externe 20 pour qu'il entoure l'outil interne, comme le montre la figure 8.

Un fois l'outil externe en place, on gonfle à nouveau le ballon 6, ce qui provoque l'expansion du manchon d'administration 22 comme le montre la figure 9, les canaux venant se plaquer contre la face interne de l'artère qui ferme ainsi la face ouverte de chaque canal. On injecte alors dans le manchon 22 la composition à administrer. Cette composition circule dans les canaux 24 et se diffuse dans toutes les ouvertures borgnes 36, ainsi que contre la face interne de l'artère. Cette étape de gonflage et d'administration dure un très court instant, sachant que la circulation du sang dans l'artère ne doit pas être interrompue trop longtemps.

Immédiatement après, on dégonfle le ballon 6 afin de rétracter le manchon 22. On procède ensuite à l'extraction du cathéter comme illustré sur la figure 10.

On expliquera plus loin quels types de composition peuvent être administrées par cette méthode. On va de suite décrire un deuxième mode de réalisation du cathéter en référence aux figures 11 à 20.

### Exemple 3 :

- les figures 11 et 12 montrent les bras et le ballon, respectivement à l'état dégonflé et gonflé, d'un cathéter selon un deuxième mode de réalisation de l'invention ;
- la figure 13 est une vue avec plus de détails des bras de la figure 11;
- La figure 14 est une vue en perspective d'un tronçon de bras du cathéter de la figure 13 ;
- La figure 15 est une vue en section transversale de l'ensemble des bras de la figure 13 ; et
- Les figures 16 à 20 illustrent des étapes de mise en oeuvre de la méthode de l'invention au moyen du cathéter des figures 11 à 15.

Dans ce mode de réalisation, les références numériques des éléments analogues sont augmentées de 100.

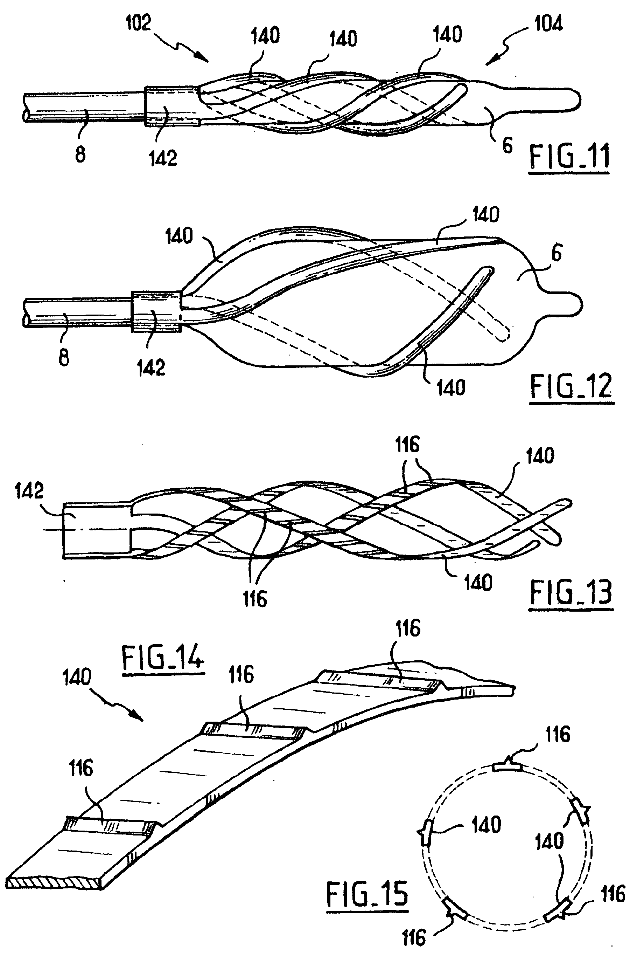

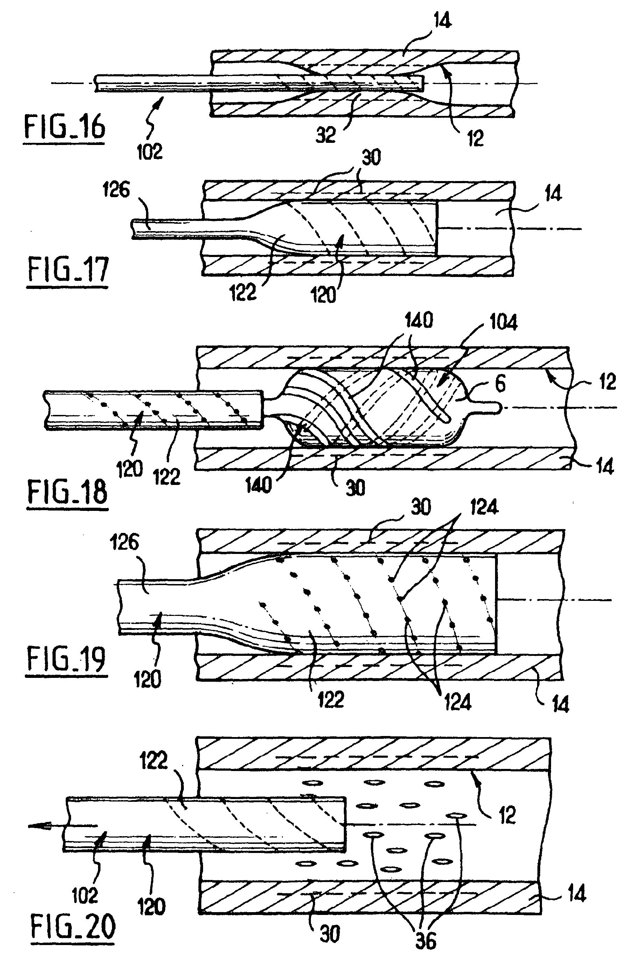

L'outil interne 104 illustré à la figure 11 comprend encore un ballon 6 monté sur un tube 8 en vue de son gonflage. L'outil interne comporte de plus des bras 140, ici au nombre de trois, portant des organes coupants. Les bras sont reliés par leur extrémité proximale à un support cylindrique commun 142 fixé au tube. Chaque bras a une forme allongée en hélice autour de l'axe du cathéter, autour du ballon. Les trois bras sont uniformément répartis autour de l'axe. Les trois bras 140 sont réalisés en un matériau élastiquement flexible. Ils sont au repos lorsque le ballon est dégonflé comme sur la figure 11. Lorsqu'on gonfle le ballon, comme sur la figure 12, les trois bras s'écartent élastiquement sous l'effet de la sollicitation du ballon. Ils conservent leur forme hélicoïdale mais le rayon de l'hélice se trouve augmenté. Chaque bras a localement une forme plate, l'épaisseur du bras s'étendant suivant la direction radiale à l'axe. Chaque bras 7 porte sur sa face externe des organes coupants constitués ici par des arêtes vives 116 s'étendant en relief et en saillie de la face externe. Chaque arête 116 est rectiligne allongée et s'étend de l'un à l'autre des bords du bras. Les arêtes sont orientées ici parallèlement à l'axe du cathéter. Toutes les arêtes sont donc parallèles entre elles et s'étendent d'avant en arrière. La figure 15 montre la disposition des arêtes et des bras dans l'hypothèse où le cathéter comporte cinq bras.

En référence aux figures 16 à 20, l'outil externe 120 du cathéter comprend encore ici un manchon creux en son centre pour pouvoir y recevoir l'outil interne 104. La paroi souple est extensible radialement et creuse suivant son épaisseur. Les deux faces interne et externe de la paroi sont continues mais la face externe présente des orifices 124 pour l'administration de la composition.

La méthode selon l'invention est mise en oeuvre à l'aide de ce cathéter de la façon suivante.

On suppose que l'on se place dans le même contexte médical que dans le premier mode de réalisation.

L'outil interne 104 se trouvant initialement à l'intérieur de l'outil externe 120, on introduit l'extrémité distale du cathéter en regard du tronçon à traiter, comme illustré à la figure 16.

En référence à la figure 17, on gonfle ensuite le ballon 6 pour étendre radialement l'ensemble du cathéter, notamment l'outil externe 120, ce qui augmente le diamètre interne de l'artère.

Le ballon demeurant à l'état gonflé, on fait coulisser l'outil externe 120 axialement vers l'arrière pour mettre les bras 140 directement en regard de l'artère, comme on le voit sur la figure 18.

On gonfle ensuite davantage le ballon pour augmenter encore son diamètre de sorte que les arêtes vives 116 entament la paroi de l'artère à partir de sa face interne et y réalisent des ouvertures borgnes 36, allongées suivant la direction longitudinale de l'artère, compte-tenu de l'orientation des arêtes vives. Les ouvertures prennent ici la forme d'incisions illustrées grossièrement à la figure 20. L'orientation de ces incisions parallèlement à la direction longitudinale de l'artère facilite l'administration de la composition.

Ensuite, en référence à la figure 19, on dégonfle partiellement le ballon et on fait coulisser sur celui-ci l'outil externe axialement vers l'avant.

En référence également à la figure 19, on gonfle à nouveau le ballon et on injecte dans l'outil externe la composition à administrer. Cette composition remplit l'épaisseur de la paroi du manchon puis s'échappe à travers les orifices 124 pour entrer en contact avec la face interne de l'artère et les ouvertures borgnes. On dégonfle ensuite le ballon et on retire le cathéter, comme sur la figure 20. Comme dans le premier mode, l'étape de l'administration de la composition a une durée très brève.

## Revendications

1. Dispositif (2 ; 102) pour administrer une composition dans une paroi d'un conduit (14) d'un corps humain ou animal, **caractérisé en ce qu'**il comprend des moyens (4 ; 104) aptes à entamer une face interne (12) de la paroi du conduit pour réaliser des ouvertures borgnes (36) dans une épaisseur de la paroi et des moyens de distribution (20 ; 120) pour mettre la composition en contact avec les ouvertures.

2. Dispositif selon la revendication 1, **caractérisé en ce que** les moyens aptes à entamer (4 ; 104) comprennent des organes de coupe (116) ou de perforation (16).

3. Dispositif selon l'une des revendications 1 à 2, **caractérisé en ce que** les moyens pour entamer (4 ; 104) sont extensibles radialement par référence à une direction axiale du dispositif.

4. Dispositif selon l'une des revendications 1 à 3, **caractérisé en ce que** les moyens pour entamer (4 ; 104) sont associés à une chambre gonflable (6 ;106).

5. Dispositif selon les revendications 2 et 4, **caractérisé en ce que** les organes de coupe ou de perforation (16) sont portés par une paroi de la chambre gonflable (6).

6. Dispositif selon la revendication 2, **caractérisé en ce que** les moyens pour entamer comprennent des bras (140) portant les organes de coupe ou de perforation (116).

7. Dispositif selon la revendication 6, **caractérisé en ce que** les bras (140) sont associés à un tube sur lequel est monté une chambre gonflable(8).

8. Dispositif selon l'une quelconque des revendications 1 à 7, **caractérisé en ce que** les moyens de distribution (120) sont extensibles radialement par référence à une direction axiale du dispositif.

9. Dispositif selon l'une quelconque des revendications 1 à 8, **caractérisé en ce que** les moyens de distribution (20) présentent des canaux (24) aptes à recevoir la composition, les canaux étant ouverts en direction opposée à un axe du dispositif ou fermés par une paroi contenant des orifices.

10. Dispositif selon l'une quelconque des revendications 1 à 9, **caractérisé en ce que** les moyens de distribution (20 ; 120) comprennent une paroi présentant des orifices externes (124).

11. Dispositif selon l'une quelconque des revendications 1 à 10, **caractérisé en ce que** les moyens de distribution (20 ; 120) sont aptes à entourer les moyens pour entamer (4 ; 104).

12. Dispositif selon l'une quelconque des revendications 1 à 11, **caractérisé en ce que** les moyens de distribution (20 ; 120) sont aptes à coulisser par rapport aux moyens pour entamer (4 ; 104) suivant une direction axiale du dispositif.

13. Dispositif selon l'une quelconque des revendications 1 à 12, **caractérisé en ce que** le ballon (4 ; 104) étend radialement les moyens de distribution (20 ; 120).

14. Dispositif selon l'une quelconque des revendications 1 à 13, **caractérisé en ce qu'**il est destiné à administrer une composition dans la paroi d'un vaisseau sanguin tel qu'une artère (14), notamment une artère portant un stent (30);

15. Dispositif selon l'une quelconque des revendications 1 à 14, **caractérisé en ce qu'**il s'agit d'un cathéter.

16. Dispositif (2 ; 102) pour administrer une composition dans une paroi d'un conduit (14) d'un corps humain ou animal, **caractérisé en ce qu'**il comprend des moyens aptes à entamer (4 ; 104) une face interne de la paroi du conduit pour réaliser des ouvertures borgnes (36) dans l'épaisseur de la paroi, ces moyens portant des'organes de coupe (116) ou de perforation (16) et étant extensibles radialement par référence à un axe du dispositif, le dispositif comportant des moyens de distribution (20 ; 120) pour mettre la composition en contact avec les ouvertures, les moyens de distribution étant extensibles radialement et aptes à entourer les moyens pour entamer (4 ; 104).
